# EUROPEAN PATENT APPLICATION

(11) **EP 2 860 476 A2**
(43) Date of publication of application: **15.04.2015**
(21) Application number: 14188439.5
(22) Date of filing: 10.10.2014
(51) Int. Cl.: F25D 3/08

(54) **Cooling gel pad**

(30) Priority: 10.10.2013 US 201314050646
(71) Applicant: Helamia LLC, New York, NY 10028 (US)
(72) Inventor: Xu, Helene, New York, NY 10028 (US)
(74) Representative: Boult Wade Tennant

(57) **Abstract**

A device for providing heat dissipation is claimed. The device comprises an outer shell and an inner, heat-dissipating gel layer within said outer shell, the inner heat-dissipating gel layer that comprises a hydrogel compound that dissipates body heat without refrigeration at room temperature.

## Description

### COPYRIGHT NOTICE

A portion of the disclosure of this patent document contains material which is subject to copyright protection. The copyright owner has no objection to the facsimile reproduction by anyone of the patent document or the patent disclosure, as it appears in the Patent and Trademark Office patent files or records, but otherwise reserves all copyright rights whatsoever.

### FIELD OF THE INVENTION

The disclosed invention relates generally to heat dissipating gel pads. More specifically, embodiments of the disclosed invention are directed towards gel pads, and methods of manufacture thereof, that allow for the independent dissipation of body heat without external requires such as refrigeration, electrical inputs, or other external assistance.

### BACKGROUND OF THE INVENTION

Frequently, seats and other surfaces are designed from a utilitarian perspective and provide little to no comfort. Common attempts to increase the comfort of surfaces focus on the tactile properties of the surface such as increasing the padding of the surface or applying various materials on top of the existing surface to increase the comfort of said surface. While these techniques may provide temporary physical comfort, many of these solutions ignore the heat dissipation from human occupants that occurs when occupants are in contact with the surfaces. For example, while wool seat covers may provide initial comfort such materials inherently trap the body heat of occupants and add to the discomfort of the occupant when in contact with the surface for extended periods of time. Further, during warmer seasons, or in warmer climates, such solutions fail to dissipate any body heat and, at times, can render the surface even more uncomfortable.

Solutions to provide cooling properties to surfaces and surface covers generally involve complicated apparatuses or undue maintenance. For example, some solutions contain constant water coolant throughout the cover which requires the attachment of a surface covering with external hoses or other connections that allow for circulation of cooling water or other coolants. Alternatively, other solutions do not require attachment of coolant supplies but require repeated refrigeration and can result in extreme variations in temperature resulting in an unpleasant shock to the occupant.

Thus, there is currently a need in the art for a cooling surface cover or pad that provides heat dissipation without the necessity of the previously discussed shortcomings. Specifically, there exists a need for a cooling gel pad that dissipates the heat from an occupant without the need for an external cooling source or repeated maintenance.

### SUMMARY OF THE INVENTION

Generally, the disclosure describes a device for providing heat dissipation. In one embodiment, the device comprises an outer shell and an inner heat-dissipating gel layer within the outer shell. In one embodiment, the inner heat-dissipating gel layer comprises a hydrogel compound that dissipates body heat without refrigeration, electrical inputs, or other external assistance.

In one embodiment the outer shell comprises a water-proof fabric shield, such as nylon taffeta fabric. Alternatively, or in conjunction with the foregoing, the outer shell further may comprise a plurality of openings. As will be discussed, the inner hydrogel may comprise a water and glycerol based compound. In further embodiments, the hydrogel may further contain one or more stabilizing agents including, but not limited to, polyacrylic acid and/or sodium chloride. In another embodiment, the hydrogel comprises 30-40% water, 40-50% glycerol, 12-18% sodium chloride and 1-4% polyacrylic acid.

The use of the hydrogel may result in the maintenance of a temperature approximately 3 to 5 degrees Celsius below room temperature. As such, the outer shell may be designed for use in strollers, car seats, or other sitting devices.

### BRIEF DESCRIPTION OF THE DRAWINGS

The invention is illustrated in the figures of the accompanying drawings which are meant to be exemplary and not limiting, in which like references are intended to refer to like or corresponding parts, and in which:
FIG. 1 illustrates a top-view of a cooling gel pad according to one embodiment of the disclosure; and
FIG. 2 illustrates a cross-sectional view of a cooling gel pad according to one embodiment of the disclosure.

### DETAILED DESCRIPTION OF THE INVENTION

In the following description of the embodiments of the disclosure, reference is made to the accompanying drawings that form a part hereof, and in which is shown by way of illustration exemplary embodiments in which the invention may be practiced. It is to be understood that other embodiments may be utilized and structural changes may be made without departing from the scope of the disclosed invention.

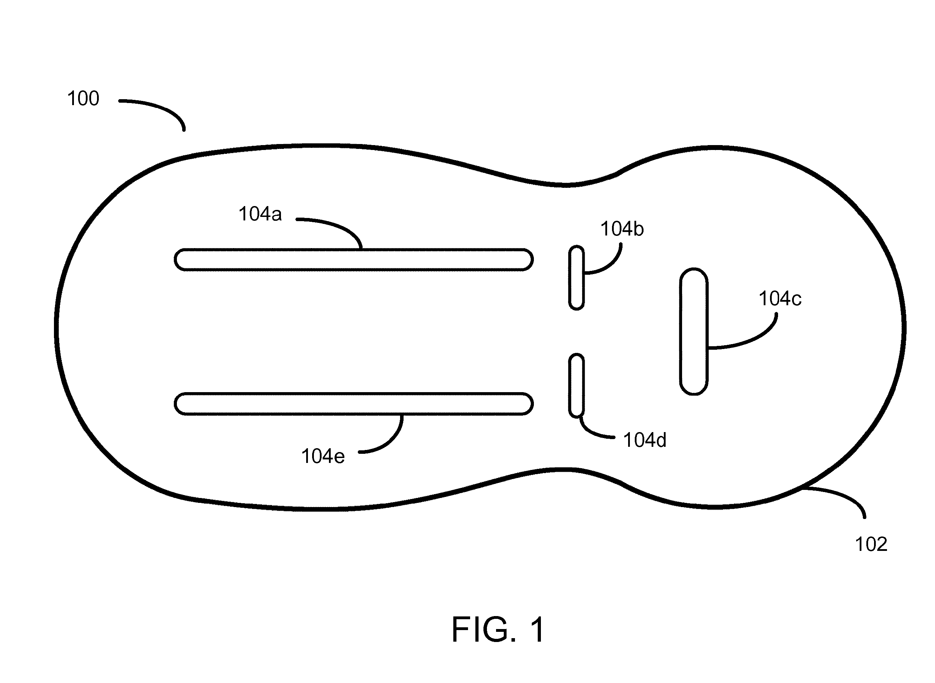

FIG. 1 illustrates a top-view of a cooling gel pad according to one embodiment of the disclosure. As illustrated in Figure 1, a gel pad 100 comprises an outer shell 102 containing a plurality of openings 104a-e.

As illustrated in Figure 1, the outer shell 102 can be designed according to the intended use of the gel pad 100. For example, the outer shell 102 in Figure 1 is designed for use within strollers or similar vehicles. Thus, the perimeter of the outer shell 102 is designed to fit within the confines of the transporting device, a stroller. Alternatively, the outer shell 102 can be designed for use in scenarios other than that of a stroller. For example, the outer shell 102 may be designed for use in automobiles, office chairs, or non-sitting uses, such as mattress pads, etc.

In one embodiment, the outer shell 102 is comprised of a fabric suitable for juxtaposition with human skin. For example, the outer shell 102 may be comprised of a water proof fabric shield. In one embodiment, the shield may comprise nylon taffeta fabric. Alternatively, or in conjunction with the foregoing, the outer shell 102 may comprise a water-proof material capable of withholding the liquid content, maintaining the integrity of the inner material discussed further herein.

As the embodiment of Figure 1 illustrates, the outer shell 102 contains a plurality of openings 102a-e. In one embodiment, the openings 102a-e comprise perforations in the outer shell 102 to allow for the insertion of straps or other restraining devices such as seatbelts. As illustrated, these openings 102a-e allow for the attachment of the gel pad 100 to other devices such as strollers, car seats, high chairs, baby bouncers or other similar devices, and allow for the gel pad 100 to remain stationary during movement of said devices.

For example, openings 104a and 104b may allow for shoulder straps of a stroller to be inserted, whereas opening 104c may allow for a waist or crotch strap to be inserted thus allowing a child to be buckled into the stroller on top of the gel pad 100. While the examples discussed herein are in the context of strollers the examples are not meant to be limited to only strollers. Alternatively, the openings 104a-e may be arranged, removed, or added in order to accommodate various carrier or other devices. For example a gel pad 100 configured for use within an automobile may contain three openings for a buckle receiver, a waist strap, and a shoulder strap as found within a traditional automobile. Further, the gel pad 100 may contain no openings when used with a device such as a mattress.

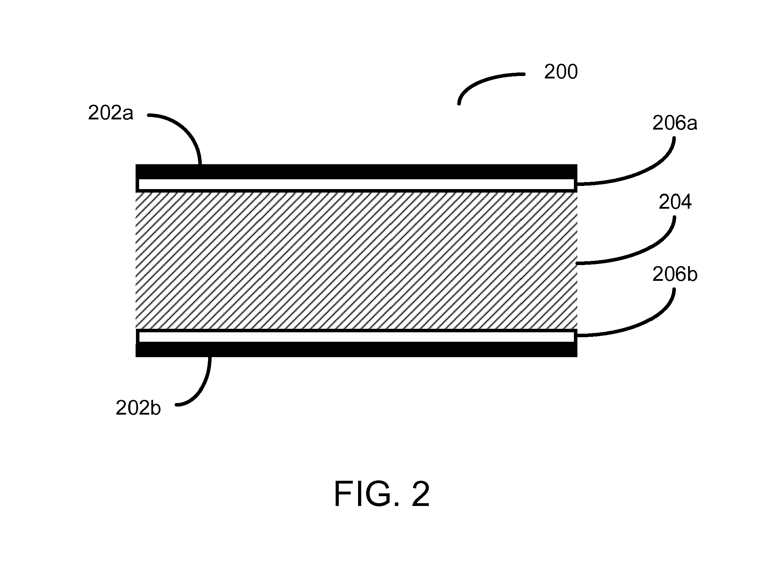

FIG. 2 illustrates a cross-sectional view of a cooling gel pad according to one embodiment of the disclosure. As illustrated in Figure 2, the gel pad 200 comprises an outer layer 202a and 202b, an inner layer 204, and a shield layer 206. The outer layers 202a and 202b may comprise an outer layer similar to that described with respect to Figure 1 and such properties are not repeated herein for the sake of clarity.

As illustrated in Figure 2, the gel pad 200 may comprise a shield layer 206a, 206b. In one embodiment, the shield layer 206a, 206b comprises a PVC shield layer that seals and isolates the inner layer 204. Use of a PVC shield layer operates to maintain the moistness of the inner layer 204 and increases the durational use of the inner layer 204. Notably, the shield layer 206a, 206b may be substantially thinner than the outer layer 202a, 202b. In some embodiments, the shield layer 206a, 206b may be fused within outer layer 202a, 202b resulting in a single, two-sided outer layer 202a, 202b.

As discussed above, an inner layer 204 is sealed within the outer layer 202a-b and provides heat dissipation to the user of the gel pad 200 without the need for external cooling sources or repeated maintenance. In one embodiment, the inner layer 204 comprises a hydrogel such as a hydrophilic cooling gel. The hydrogel primary consists of water and includes additional elements to act as stabilizing agents in order to provide a gel-like texture. Thus, the inner layer 204 comprises a flexible gel-like texture to provide both thickness and flexibility. By utilizing the gel-like properties of the hydrogel, the gel pad 200 is able to provide both the beneficial cooling effects as well as comforting effects provided by the gel.

As discussed above, the inner layer 204 contains a certain percentage of water. The use of water allows for the quick absorption and dissipation of body heat from the user of the pad. In contrast to previous techniques however, the inner layer 204 further contains stabilizing agents within the inner layer 204 and intermixed with the water. These agents substantially increase the speed of cooling and enhance the power of said cooling. In one embodiment, the hydrogel contains less than fifty percent water by volume. Additionally, the hydrogel may contain a combination of chemicals in addition to the water including, but not limited to, glycerol, sodium chloride, and polyacrylic acid. In one embodiment, the chemical combination is as follows:

**TABLE 1**

| | |
|---|---|
| Water | 30-40% |
| Glycerol | 40-50% |
| Sodium Chloride | 12-18% |
| Polyacrylic acid | 1-4% |

Notably, however various percentages of the above combination maybe altered and the above example is not intended to be limited. Further, comparable chemicals may be substitute for the above listed chemicals if appropriate.

The above described chemical compositions provide substantial benefits not found in the current state of the art. For example, when the temperature of the environment is between 5 to 35 degrees Celsius, the temperature of the pad remains generally between 2 to 5 degrees Celsius below the temperature of the environment. Thus, as can be seen, under reasonable conditions the hydrogel maintains a temperature that is consistently cooler than the outdoor temperature, even as the outdoor temperature fluctuates. Furthermore, while not required, the gel pad 200 may be refrigerated in order to extend the duration of the cooling effect if the outdoor temperature is above room temperature. Notably, however, the gel pad 200 remains operational despite a lack of refrigeration, electrical inputs, or other external assistance.

FIGS. 1-2 are conceptual illustrations allowing for an explanation of the disclosed invention. Notably, the figures and examples above are not meant to limit the scope of the disclosed invention to a single embodiment, as other embodiments are possible by way of interchange of some or all of the described or illustrated elements. Moreover, where certain elements of the disclosed invention can be partially or fully implemented using known components, only those portions of such known components that are necessary for an understanding of the disclosed invention are described, and detailed descriptions of other portions of such known components are omitted so as not to obscure the invention. In the present specification, an embodiment showing a singular component should not necessarily be limited to other embodiments including a plurality of the same component, and vice-versa, unless explicitly stated otherwise herein. Moreover, applicants do not intend for any term in the specification or claims to be ascribed an uncommon or special meaning unless explicitly set forth as such. Further, the disclosed invention encompasses present and future known equivalents to the known components referred to herein by way of illustration.

The foregoing description of the specific embodiments so fully reveal the general nature of the invention that others can, by applying knowledge within the skill of the relevant art(s) (including the contents of the documents cited and incorporated by reference herein), readily modify and/or adapt for various applications such specific embodiments, without undue experimentation, without departing from the general concept of the disclosed invention. Such adaptations and modifications are therefore intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one skilled in the relevant art(s).

While various embodiments of the disclosed herein have been described above, it should be understood that they have been presented by way of example, and not limitation. It would be apparent to one skilled in the relevant art(s) that various changes in form and detail could be made therein without departing from the spirit and scope of the invention. Thus, the disclosed invention should not be limited by any of the above-described exemplary embodiments, but should be defined only in accordance with the following claims and their equivalents.

## Claims

1. A device for providing heat dissipation, the device comprising:
an outer shell; and
an inner, heat-dissipating gel layer within said outer shell, the inner heat-dissipating gel layer comprising a hydrogel compound that dissipates body heat without refrigeration at room temperature.

2. The device of claim 1 wherein the outer shell comprises a water-proof fabric shield.

3. The device of claim 2 wherein the water-proof fabric comprises nylon taffeta.

4. The device of claim 1 wherein the outer shell further comprises a plurality of openings.

5. The device of claim 1 wherein the hydrogel compound comprises a water and glycerol based compound.

6. The device of claim 5 wherein the hydrogel further comprises one or more stabilizing agents.

7. The device of claim 6 wherein the stabilizing agents comprise polyacrylic acid and sodium chloride.

8. The device of claim 7 wherein the hydrogel comprises 30-40% water, 40-50% glycerol, 12-18% sodium chloride and 1-4% polyacrylic acid.

9. The device of claim 1 wherein the outer shell is designed for use with a sitting device.

10. The device of claim 9 wherein the sitting device comprises a stroller.

11. The device of claim 9 wherein the sitting device comprises a car seat.

12. The device of claim 1 wherein the hydrogel maintains a constant temperature without electrical input or other external assistance.

13. The device of claim 1 wherein the hydrogel maintains a temperature approximately 2 to 5 degrees Celsius below the temperature of the outdoor environment.

14. The device of claim 1 further comprising a shield layer located between the outer shell and inner layer.
